# EUROPÄISCHE PATENTSCHRIFT

(11) **EP 0 361 086 B1**
(45) Veröffentlichungstag und Bekanntmachung des Hinweises auf die Patenterteilung: **22.09.1993**
(21) Anmeldenummer: 89115624.2
(22) Anmeldetag: 24.08.1989
(51) Int. Cl.: A61B 1/12

(54) **Einrichtung zur Versorgung einer Körperhöhle mit einer Spülflüssigkeit über ein Endoskop**
Fluid irrigation supply means of a cavity of the body using an endoscope
Dispositif d'alimentation de liquides de rinçage dans une cavité du corps par un endoscope

(30) Priorität: 17.09.1988 DE 3831661
(43) Veröffentlichungstag der Anmeldung: 04.04.1990
(73) Patentinhaber: Richard Wolf GmbH, 75438 Knittlingen (DE)
(72) Erfinder: Schäfer, Roland, D-7518 Bretten-Diedelsheim (DE); Ams, Felix, D-7539 Kämpfelbach (DE)
(74) Vertreter: Wilcken, Hugo

(56) Entgegenhaltungen:
- EP-A- 0 029 556
- WO-A-82/03545
- DE-C- 3 716 230

## Beschreibung

Die Erfindung geht aus von einer Einrichtung zum Versorgen einer Körperhöhle über einen Endoskopkanal mit einer Spülflüssigkeit aus einem mittels einer ersten steuerbaren Pumpe unter Luftdruck setzbaren Flüssigkeits vorratsbehälter und zum Absaugen der Spülflüssigkeit und Körpersekreten oder dergleichen aus der Körperhöhle in einen mittels einer zweiten steuerbaren Pumpe an Unterdruck legbaren Sammelbehälter.

Bei dieser bekannten Einrichtung ist es möglich, die Flüssigkeitszufuhr in die Körperhöhle einzuleiten und die Zufuhr zu unterbrechen. Nach jeder Unterbrechung können geringe Restmengen der Spülflüssigkeit aus der distalen Öffnung des durch das Endoskop verlaufenden Kanals unkontrolliert austreten und dadurch das Optikausblickfenster überfluten und die Sicht des Benutzers wesentlich beeinträchtigen. Ein weiterer Nachteil der bekannten Einrichtung besteht darin, daß bei beginnendem Absaugen der Spülflüssigkeit aus der Körperhöhle sofort der Unterdruck der Absaugpumpe in voller Höhe einsetzt, so daß das distale Ende des Endoskops, sofern es sich im Bereich einer Körperhöhlenwand oder eines Organs befindet, daran anhaftet, was eine Unterbrechung des Absaugvorganges und die erneute Durchführung des Spülvorganges erfordert.

Die Aufgabe der Erfindung besteht darin, das Verbleiben von Restmengen der Spülfüssigkeit im durch das Endoskop verlaufenden Kanal und das unkontrollierte Austreten in die Körperhöhle wirksam zu verhindern und das Anhaften des distalen Endoskopkanalendes an Körperhöhlenwänden zu unterbinden.

Diese Aufgabe wird durch die Merkmale des Anspruches 1 gelöst.

Durch diese Lösung ist es möglich, nach jeder Unterbrechung der Spülflüssigkeitszufuhr in die Körperhöhle auf den durch das Endoskop verlaufenden Kanal einen geringen Mindestunterdruck auszuüben, der ausreicht, um die im Kanal verbleibende, verhältnismäßig geringe Restflüssigkeitsmenge in einen Sammelbehälter abzusaugen, wobei durch diesen Mindestunterdruck auch das Anhaften des distalen Endoskopendes an Körperhöhlenwandungen vermieden wird. Dieser anfängliche Mindestunterdruck erhöht sich sodann automatisch für das Absaugen der Spülflüssigkeit aus der Körperhöhle auf den erforderlichen einstellbaren Sollwert.

Es ist nach der DE-C-34 15 837 bekannt, bei einer optischen Untersuchung mittels eines Endoskopes zu verhindern, daß unter Umständen während einer Untersuchung einer Körperhöhle eines Patienten durch versehentliches Handbetätigen einer Einrichtung eine Flüssigkeitszufuhr in die Körperhöhle erfolgen kann. Diese Aufgabe und Lösung stimmen nicht mit der der Erfindung zugrundeliegenden Aufgabe und Lösung überein.

Nach der DE-A-3 803 212.0 ist es weiter bekannt, bei Endoskopen für die nasale Chirurgie für den Saug- und Spülschaft eines Endoskops ein von Hand betätigbares Umschaltventil für die Verbindung mit einer Unterdruckquelle bzw. Spülmittelquelle und für die Unterbrechung dieser Verbindung vorzusehen. Auch diese Lösung ist nicht geeignet, das Verbleiben von Restmengen von Spülflüssigkeiten im Endoskopkanal zu verhindern.

Aus der EP-A-0 029 556 ist ein Endoskopsystem bekannt, das ein Endoskop mit Saug- und Spülkanälen für Luft und Wasser sowie Pumpen zum Durchleiten von Luft und Wasser durch die entsprechenden Kanäle aufweist. Die Pumpen erzeugen entsprechend den von einer Kontrolleinheit bestimmten Steuersignalen die Fließrate für die Luft und das Wasser. Die Fließrate kann zwar bei diesem System niedrig eingestellt werden, womit aber nur beim Absaugen geringer Mengen von Blut oder Flüssigkeit aus einer Körperhöhle verhindert wird, daß das distale Ende des Endoskopes unter der Wirkung des Unterdruckes der Absaugpumpe an der Wand der Körperhöhle anhaftet bzw. die Körperhöhlenwand angesaugt wird. Ferner ist mit diesem System ein unkontrolliertes Austreten geringer Restmengen von Spülflüssigkeit aus der distalen Öffnung eines durch das Endoskop verlaufenden Kanales nicht verhinderbar.

Vorteilhafte Ausgestaltungen der Erfindung sind in den Unteransprüchen aufgeführt.

Die Erfindung ist nachstehend anhand der anliegenden Zeichnung beispielsweise erläutert.

Das Endoskop 1 ist in bekannter Weise von einem Spül- und Saugkanal durchlaufen. An das Steuergehäuse 1a des Endoskops schließen sich u.a. Leitungen 2, 4 an, die an einem in einem Flüssigkeits-Vorratsbehälter 5 befindlichen Steigrohr angeschlossen sind, wobei der Behälter mit Druckluft beschickbar ist. Die Luft wird über eine Leitung 12, über ein Sterilfilter 13, durch eine erste, von einer einschaltbaren elektrischen oder elektronischen Einheit 11 in Berieb setzbaren Pumpe 14, ein Ventil 17 und über die Leitungen 18 und 3 als Druckluft mit einem kontinuierlich erreichten Sollwert dem Vorratsbehälter 5 zugeführt, so daß die Flüssigkeit aus dem Behälter 5 über das Steigrohr und die Leitungen 2, 4 und den Endoskopkanal in die Körperhöhle geleitet wird. Nach Öffnen eines Fuß- oder Tastschalters der Einheit 11 kann die Flüssigkeitszufuhr jederzeit unterbunden werden. Dabei wird gleichzeitig der Behälter 5 über die Leitungen 3 und 18 und durch ein Ventil 19 drucklos gemacht, wobei die Zeit zum Abbau des Druckes im Behälter 5 durch die Einheit 11 eingestellt werden kann.

Da nun noch eine Restflüssigkeit im Spülkanal des Endoskops ansteht, die in die Körperhohle mit Sichtbehinderung austreten kann, ist in die Leitungen 6, 4, 2 ein Umschaltventil 26 eingegliedert, durch dessen Betätigung mittels der Steuereinheit 11 die Leitung 2 von der Leitung 4 ab- und an die bei 9 abklemmbare Saugleitung 6 angeschlossen wird. Diese Saugleitung endet in einem Sammelbehälter 8, der über eine Leitung 7, einen Anschluß 20 und eine Leitung 25 an die Saugseite einer zweiten Pumpe 27 angeschlossen ist. Zwischen dem Anschluß 20 und der Pumpe 27 ist ein Hydrophob-Filter 21 geschaltet, um zu verhindern, daß in die innerhalb der Versorgungseinheit angeordneten Schlauchleitungen Flüssigkeit, Sekret oder dergleichen eindringen und dadurch eine Kontaminierung erfolgen kann.

Das Absaugen der Restflüssigkeit aus dem Endoskopkanal erfolgt automatisch nach jeder Spülflüssigkeitszufuhr durch die Steuereinheit 11.

Durch den Betrieb der zweiten Pumpe 27 wird also die Restflüssigkeit im Endoskopkanal in den Sammelbehälter 8 gesaugt. Damit diese Pumpe 27 nicht sofort mit dem maximalen Sollwert des Pumpenunterdruckes arbeitet; um das Anlegen des distalseitigen Endoskopendes beispielsweise an einer Körperhöhlenwandung zu vermeiden, ist ein Regler 22 vorgesehen, der ein Regelventil 23 in der Saugleitung 25 so betätigt, daß der durch die Pumpe 27 erzeugte Unterdruck zunächst im Sammelbehälter 8 einen geringen Mindestwert erreicht, durch den es zwar möglich ist, die Flüssigkeitsrestmenge im Endoskopkanal abzusaugen, nicht aber dazu führen kann, daß das distale Endoskopende an einer Körperhöhlenwandung anhaftet. Der zum Absaugen erforderliche Unterdruck wird durch den Regler 22, der das Ventil 23 ansteuert, allmählich auf den vorwählbaren Sollwert erhöht. Nach dem Absaugen der Spülflüssigkeit wird ein vom Regler 22 angesteuertes Ventil 24 so weit geöffnet, daß der Unterdruck im Sammelbehälter 8 auf einen geringen Mindestwert abgesenkt wird. Dadurch wird bei einem erneuten Absaugvorgang ein unbeabsichtigtes Anhaften des distalen Endoskopendes an einer Körperhöhlenwandung vermieden, wie es vorstehend beschrieben ist.

Zum Entleeren des Sammelbehälters 8 wird das Ventil 24 durch die Steuereinheit 11 betätigt, bis der Sammelbehälter völlig drucklos wird, so daß der Behälter 8 leicht geöffnet werden kann. Dieser Vorgang kann wahlweise manuell zu jedem beliebigen Zeitpunkt oder automatisch zum Zeitpunkt der Außerbetriebnahme der Versorgungseinheit eingeleitet werden.

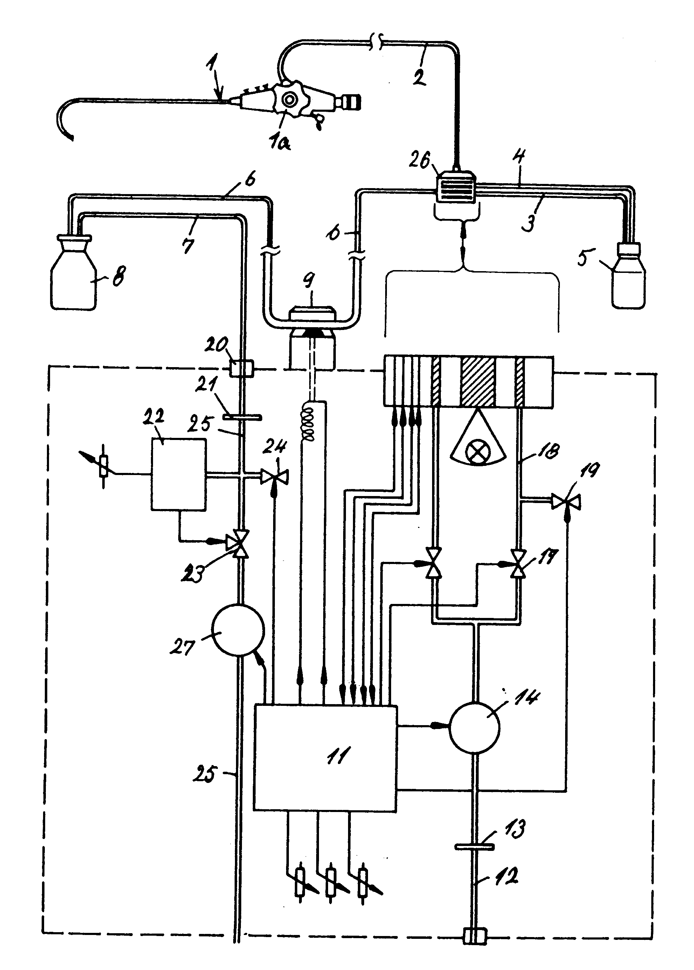

## Patentansprüche

1. Einrichtung zum Versorgen einer Körperhöhle über einen Endoskopkanal mit einer Spülflüssigkeit aus einem mittels einer ersten steuerbaren Pumpe (14) unter Luftdruck setzbaren Flüssigkeitsvorratsbehälter (5) und zum Absaugen der Spülflüssigkeit und Körpersekreten oder dergleichen aus der Körperhöhle in einen mittels einer zweiten steuerbaren Pumpe (27) an Unterdruck legbaren Sammelbehälter (8), dadurch gekennzeichnet, daß eine elektrische oder elektronische Steuereinheit (11) vorgesehen ist, die die erste Pumpe (14) bis zum Erreichen eines auf einen Sollwert kontinuierlich ansteigenden Überdruckes im Vorratsbehälter (5) anschaltet, den Vorratsbehälter nach jeder Spülflüssigkeitszufuhr aus dem Behälter (5) in einer vorwählbaren Zeit über ein Ventil (19) entlüftet und daß anschließend durch die Steuereinheit (11) ein Umschaltventil (26) betätigt wird, durch das der Spül- und Insufflationskanal im Endoskop (1) über eine Saugleitung (6) an den durch die zweite Pumpe (27) unter Unterdruck setzbaren Sammelbehälter (8) angeschlossen wird, und daß ein Regler (22) den durch die zweite Pumpe (27) erzeugten Unterdruck durch ein Regelventil (23) von einem Mindestwert kontinuierlich auf einen vorwählbaren Sollwert ansteigen läßt und den Sammelbehälter (8) nach jedem Absaugvorgang durch Öffnen eines Ventils (24) auf den Mindestwert belüftet.

2. Einrichtung nach Anspruch 1, dadurch gekennzeichnet, daß der Sammelbehälter (8) zum Entleeren über das von der Steuereinheit (11) betätigbare Ventil (24) belüftbar ist.

3. Einrichtung nach Anspruch 1 oder 2, dadurch gekennzeichnet, daß in der Verbindungsleitung (7, 25) zwischen dem Sammelbehälter (8) und der zweiten Pumpe (27) ein Hydrophob-Filter (21) angeordnet ist.

## Claims

1. Equipment for supplying a body cavity by way of an endoscope duct with a flushing liquid from a liquid supply container (5) which can be placed under air pressure by means of a first controllable pump (14) and for sucking the flushing liquid and body secretions or the like away from the body cavity into a collecting container (8) which can be subjected to underpressure by means of a second controllable pump (27), characterized in that an electrical or electronic control unit (11) is provided which switches the first pump (14) on until an excess pressure rising continuously to a desired value is reached in the supply container (5), vents the supply container after each supply of flushing liquid from the container (5) in a preselectable time by way of a valve (19) and that then through the control unit (11) a change-over valve (26) is actuated by which the flushing and insufflation duct in the endoscope (1) is connected by way of a suction pipe (6) to the collecting container (8) which can be placed under underpressure by the second pump (27), and in that a regulator (22) allows the underpressure, generated by the second pump (27), through a regulating valve (23) to rise from a minimum value continuously to a preselectable desired value and after each suction procedure ventilates the collecting container (8) to the minimum value by opening a valve (24).

2. Equipment according to claim 1, characterised in that the collecting container (B) can be ventilated for emptying by way of the valve (24) which can be actuated by the control unit (11).

3. Equipment according to claim 1 or 2, characterised in that a hydrophobic filter (21) is arranged in the connecting pipe (7, 25) between the collecting container (8) and the second pump (27).

## Revendications

1. Dispositif pour alimenter une cavité corporelle par l'intermédiaire d'un canal d'endoscope, avec un liquide de lavage à partir d'un réservoir de liquide (5) pouvant être soumis à une pression d'air au moyen d'une première pompe pouvant être commandée (14), et pour aspirer le liquide de lavage et les sécrétions corporelles ou analogues à partir de la cavité corporelle pour les introduire dans un réservoir de collecte (8) pouvant être placé en dépression au moyen d'une seconde pompe pouvant être commandée (27), caractérisé en ce qu'il est prévu une unité de commande électrique ou électronique (11), qui raccorde la première pompe (14) jusqu'à ce qu'une surpression, qui augmente continûment jusqu'à une valeur de consigne, soit atteinte dans le réservoir (5), désaère le réservoir pendant un intervalle de temps pouvant être présélectionné, par l'intermédiaire d'une soupape (19), après chaque envoi d'un liquide de lavage à partir du réservoir (5), et en ce qu'ensuite l'unité de commande (11) actionne une soupape de commutation (26), au moyen de laquelle le canal de lavage et d'insufflation dans l'endoscope (1) est raccordé par l'intermédiaire d'une conduite d'aspiration (6) au réservoir de collecte (8) pouvant être placé en dépression par l'intermédiaire de la seconde pompe (27), et en ce qu'un régulateur (22) fait croître continûment, depuis une valeur minimale jusqu'à une valeur de consigne pouvant être préprésélectionnée, la dépression produite par la seconde pompe (27), au moyen d'une soupape de régulation (23) et aère le réservoir de collecte (8), jusqu'à la valeur minimale, après chaque opération d'aspiration, par ouverture d'une soupape (24).

2. Dispositif selon la revendication 1, caractérisé en ce que le réservoir de collecte (8) peut être aéré pour le vidage, par l'intermédiaire de la soupape (24) pouvant être actionnée par l'unité de commande (11).

3. Dispositif selon la revendication 1 ou 2, caractérisé en ce qu'un filtre hydrophobe (21) est disposé dans la conduite de liaison (7,25) entre le réservoir de collecte (8) et la seconde pompe (27).
